# EUROPEAN PATENT SPECIFICATION

(11) **EP 1 643 985 B1**
(45) Date of publication and mention of the grant of the patent: **23.02.2011**
(21) Application number: 04738961.4
(22) Date of filing: 29.06.2004
(51) Int. Cl.: A61K 31/16, A61K 31/343, A61K 31/138, A61K 31/135, A61P 25/24

(54) **THE COMBINATION OF A SEROTONIN REUPTAKE INHIBITORS AND AGOMELATINE**
KOMBINATION VON SEROTONIN-WIEDERAUFNAHME-HEMMERN UND AGOMELATIN
COMBINAISON D'UN INHIBITEUR DE RECAPTAGE DE LA SEROTONINE ET D'AGOMELATINE

(30) Priority: 04.07.2003 DK 200301031; 07.07.2003 US 485479 P
(43) Date of publication of application: 12.04.2006
(73) Proprietor: H. Lundbeck A/S, 2500 Valby (DK)
(72) Inventor: WILLIGERS, Sandra, DK-2880 Bagsværd (DK)
(74) Representative: Conrad, Lars Sparre
(86) International application number: PCT/DK2004/000464
(87) International publication number: WO 2005/002562

(56) References cited:
- EP-A- 0 447 285
- EP-A- 0 714 663
- CHILMAN-BLAIR K ET AL: "Agomelatine. Antidepressant treatment of bipolar disorder, melatonin agonist/5-HT2C antagonist." DRUGS OF THE FUTURE, vol. 28, no. 1, January 2003 (2003-01), pages 7-13, XP009036058 ISSN: 0377-8282
- BOURIN MICHEL ET AL: "Antidepressant-like activity of S 20098 (agomelatine) in the forced swimming test in rodents: involvement of melatonin and serotonin receptors." JOURNAL OF PSYCHIATRY & NEUROSCIENCE : JPN. MAR 2004, vol. 29, no. 2, March 2004 (2004-03), pages 126-133, XP009036056 ISSN: 1180-4882
- PAPP MARIUSZ ET AL: "Effect of agomelatine in the chronic mild stress model of depression in the rat." NEUROPSYCHOPHARMACOLOGY : OFFICIAL PUBLICATION OF THE AMERICAN COLLEGE OF NEUROPSYCHOPHARMACOLOGY. APR 2003, vol. 28, no. 4, April 2003 (2003-04), pages 694-703, XP009036057 ISSN: 0893-133X
- MONTGOMERY S A ET AL: "TREATMENT OF DEPRESSION WITH ASSOCIATED ANXIETY: COMPARISONS OF TRICYCLIC ANTIDEPRESSANTS AND SELECTIVE SEROTONIN REUPTAKE INHIBITORS" ACTA PSYCHIATRICA SCANDINAVICA, MUNKSGAARD, COPENHAGEN, DE, vol. 101, no. SUPPL 403, 2000, pages 9-16, XP008012233 ISSN: 0001-690X
- MASAND P S ET AL: "SELECTIVE SEROTONIN-REUPTAKE INHIBITORS: AN UPDATE" HARVARD REVIEW OF PSYCHIATRY, ST. LOUIS, MO, US, vol. 7, no. 2, 1 January 1999 (1999-01-01) , pages 69-84, XP001028620 ISSN: 1067-3229

## Description

The present invention relates to the use of a combination of agomelatine and an SSRI, for the treatment of depression and other affective disorders.

### Background

Selective serotonin reuptake inhibitors (hereinafter referred to as SSRIs) have become first choice therapeutics in the treatment of depression, certain forms of anxiety and social phobias, because they are effective, well tolerated and have a favourable safety profile compared to the classic tricyclic antidepressants. E.g. Montgomery and Judge. Acta Psychiatr. Scand. 2000, 101 (suppl. 403), p 9-16 describes a review study comparing TCAs and SSRIs and concludes that considering the overall risk:benefit ratio, SSRIs should be the first line treatment for depression with associated anxiety.

However, clinical studies on depression and anxiety disorders indicate that non-response to SSRIs is substantial, up to 30%. Another, often neglected, factor in antidepressant treatment is compliance, which has a rather profound effect on the patient's motivation to continue pharmacotherapy.

First of all, there is the delay in therapeutic effect of SSRIs. Sometimes symptoms even worsen during the first weeks of treatment. Secondly, sexual dysfunction is a side effect common to all SSRIs. Without addressing these problems, real progress in the pharmacotherapy of depression and anxiety disorders is not likely to happen.

In order to cope with non-response, psychiatrists sometimes make use of augmentation strategies. Augmentation of antidepressant therapy may be accomplished through the co-administration of mood stabilizers such as lithium carbonate or triiodothyronin or by the use of electroshock.

In 1993, an augmentation strategy with pindolol was described by Artigas et al. in Trends Pharmacol. Sci. 1993, 14, p 262-263. Artigas' idea is based on intracerebral microdialysis experiments in animals. In fact, later neurochemical studies built on the desensitization hypothesis by Blier and co-workers stated that the delay in therapeutic effect of antidepressants is related to a gradual desensitization of 5-HT autoreceptors (Blier et al. J. Clin. Psycipharmacol. 1987, 7 suppl. 6, 24S-35S). A key point in their hypothesis is that the effects of SSRIs on the release-controlling somatodendritic autoreceptors (5-HT_{1A}) limit the release of 5-HT in terminal areas and thus the effect of 5-HT uptake inhibition in those regions. This is supported by microdialysis experiments in rats, showing that the increase in extracellular 5-HT elicited by a single dose of an SSRI is augmented by co-administration of a 5-HT_{1A} autoreceptor antagonist (Invernizzi et al. Brain Res, 1992, 584, p322-324 and Hjorth, S., J. Neurochem, 1993, 60, p 776-779).

The effect of combined administration of a compound that inhibits serotonin reuptake and a 5-HT_{1A} receptor antagonist has been evaluated in several studies (Innis, R.B. et al. Eur. J. Pharmacol. 1987, 143, p. 1095-204 and Gartside, S.E., Br. J. Pharmacol, 1995, 115, p 1064-1070, Blier, P. et al. Trends in Pharmacol. Science 1994, 15, 220). In these studies it was found that 5-HT_{1A} receptor antagonists would abolish the initial brake on 5-HT neurotransmission induced by the serotonin reuptake inhibitors and thus produce an immediate boost of 5-HT transmission and a rapid onset of therapeutic action.

Several patent applications have been filed which cover the use of a combination of a 5-HT_{1A} antagonist and a serotonin reuptake inhibitor for the treatment of depression (see e.g. EP-A2-687 472 and EP-A2-714 663).

Another approach to increase terminal 5-HT would be through blockade of the 5-HT_{1B} autoreceptor. Microdialysis experiments in rats have indeed shown that increase of hippocampal 5-HT by citalopram is potentiated by GMC 2-29, an experimental 5-HT_{1B} receptor antagonist.

Several patent applications covering the combination of an SSRI and a 5-HT_{1B} antagonist or partial agonist have also been filed (WO 97/28141, WO 96/03400, EP-A-701819 and WO 99/13877).

Chilman-Blair et al. Drugs of the Future 2003, 28 (1), p 7-13 reports that agomelatine is a melatonin analogue that has antidepressant properties associated with the normalization of circadian rhythm patterns. Agomelatine is described as having high affinity and agonist potency at the melatonin receptor. Agomelatine is also described as having antagonistic potency at the 5-HT_{2C} receptor. The investigators report that agomelatine reduces immobility in the Forced Swim Test (FST) following acute and repeated administration.

Papp et al. Neuropsychopharmacol. 2003, 28 (4), p 694-703 studied the action of agomelatine in a Chronic Mild Stress (CMS) model to determine the involvement of circadian rhythms by administering the compound before or after dark phase. The investigators compared the activity of agomelatine to traditional antidepressants, imipramine and fluoxetine and to a melatonin receptor antagonist. Agomelatine was shown to be active in the CMS model.

EP 0447285 A discloses the synthesis and potential uses of agomelatine and other derivatives. Various pharmacological properties are described including anxiolytic activities, increased microcirculation, stimulated the immune system, inhibition of ovulation, demonstrated analgesic properties, induced sleep, binding to the melatonin receptor, and decreased blood glucose.

It has now surprisingly been found that agomelatine having the general formula 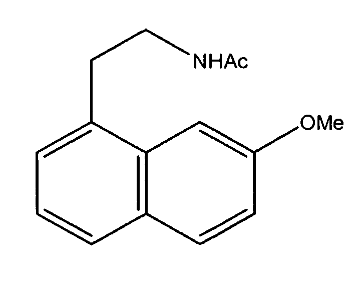 or pharmaceutically acceptable salts thereof may be used to augment and provide faster onset of the therapeutic effect of serotonin reuptake inhibitors.

### The invention

The present invention thus provides:

The present invention relates to the use of agomelatine or a pharmaceutically acceptable salt thereof and a compound, which is an SSRI for the preparation of a pharmaceutical composition or kit.

In particular, the present invention relates to the use as above, of agomelatine, or a pharmaceutically acceptable salt thereof and a compound, which is an SSRI, for the preparation of a pharmaceutical composition or kit for the treatment of depression, anxiety disorders and other affective disorders, eating disorders such as bulimia, anorexia and obesity, phobias, dysthymia, premenstrual syndrome, cognitive disorders, impulse control disorders, attention deficit hyperactivity disorder and drug abuse, in particular depression.

The anxiety disorders mentioned above include general anxiety disorder, panic anxiety, obsessive compulsive disorder, acute stress disorder, post trauma stress disorder or social anxiety disorder.

In one embodiment, the present invention relates to the use of agomelatine or a pharmaceutically acceptable salt thereof for the preparation of a pharmaceutical composition as above, which is adapted for simultaneous administration of the active ingredients. In particular, such pharmaceutical compositions may contain the active ingredients within the same unit dosage form, e.g. in the same tablet or capsule. Such unit dosage forms may contain the active ingredients as a homogenous mixture or in separate compartments of the unit dosage form.

In another embodiment, the present invention relates to the use of agomelatine or a pharmaceutically acceptable salt thereof for the preparation of a pharmaceutical composition or kit as above, which is adapted for sequential administration of the active ingredients. In particular, such pharmaceutical compositions may contain the active ingredients in discrete unit dosage forms, e.g. discrete tablets or capsules containing either of the active ingredients. These discrete unit dosage forms may be contained in the same container or package, e.g. a blister pack.

As used herein the term kit means a pharmaceutical composition containing each of the active ingredients, but in discrete unit dosage forms.

The invention also relates to a pharmaceutical composition or kit comprising agomelatine or a pharmaceutically acceptable salt thereof and a compound, which is an SSRI, and optionally pharmaceutically acceptable carriers or diluents.

The pharmaceutical composition or kit of the invention may be adapted for simultaneous administration of the active ingredients or for sequential administration of the active ingredients, as described above.

Finally, the present invention relates to agomelatine or a pharmaceutically acceptable salt thereof and an SSRI for use in the treatment of treatment of depression, anxiety disorders and other affective disorders, eating disorders such as bulimia, anorexia and obesity, phobias, dysthymia, premenstrual syndrome, cognitive disorders, impulse control disorders, attention deficit hyperactivity disorder and drug abuse, in particular depression.

As mentioned above, anxiety disorder includes general anxiety disorder, panic anxiety, obsessive compulsive disorder, acute stress disorder, post trauma stress disorder or social anxiety disorder.

Agomelatine and the SSRI may be administered simultaneously as described above.

Alternatively, the active ingredients may be administered sequentially, e.g. in two discrete unit dosage forms as described above.

Agomelatine and pharmaceutically acceptable salts thereof having the general formula 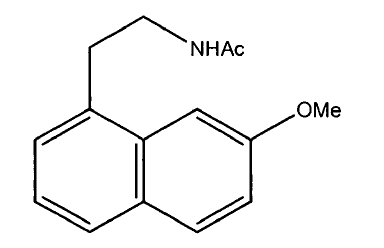 are covered by EP-B 1-447285. The compounds claimed therein are described as having affinity for the melatonin receptor.

It has now, surprisingly, been found that co-administration of agomelatine and an SSRI produces a significant increase in the level of serotonin in terminal areas, as measured in microdialysis experiments, compared to the administration of the SSRI alone. Administration of agomelatine alone causes no increase in serotonin levels as measured in microdialysis experiments.

As mentioned above, SSRIs show delayed onset of action. Even in responders to SSRIs, several weeks of treatment are necessary to achieve a relief in symptoms. Agomelatine may provide fast onset of therapeutic effect of SSRIs.

The use of a combination of agomelatine and an SSRI may greatly reduce the amount of SSRI necessary to treat depression and other affective disorders and may thus reduce the side effects caused by the SSRI. In particular, the combination of a reduced amount of SSRI and agomelatine may reduce the risk of SSRI-induced sexual dysfunction and sleep disturbances.

Co-administration of agomelatine and an SSRI may also be useful for the treatment of refractory depression, i.e. depression, which cannot be treated appropriately by administration of a serotonin reuptake inhibitor alone. Typically, agomelatine may be used as add-on therapy for the augmentation of the response to SSRIs in patients where at least 40-60% reduction in symptoms has not been achieved during the first 6 weeks of treatment with an SSRI.

As used herein, the term augmenting covers improving the therapeutic effect and/or potentiating the therapeutic effect of an SSRI.

Many antidepressants with serotonin reuptake inhibiting effect have been described in the literature. Any pharmacologically active compound which primarily or partly exert its therapeutic effect via selective inhibition of serotonin reuptake in the CNS, may benefit from augmentation with agomelatine.

The term selective serotonin reuptake inhibitor (SSRI) means an inhibitor of the monoamine transporters which has stronger inhibitory effect at the serotonin transporter than the dopamine and the noradrenaline transporters.

The following list contains a number of SSRIs, which may benefit from augmentation with agomelatine: citalopram, escitalopram, fluoxetine, fluvoxamine, sertraline or paroxetine. The compounds mentioned above may be used in the form of the base or a pharmaceutically acceptable acid addition salt thereof.

The above list of SSRIs may not be construed as limiting.

The active ingredients according to the invention, i.e. agomelatine and the SSRI, may be used in the free base form or in the form of a pharmaceutically acceptable acid addition salt thereof, the latter being obtainable by reaction of the base form with an appropriate acid.

Citalopram is preferably used in the form of the hydrobromide or as the base, escitalopram in the form of the oxalate, fluoxetine, sertraline and paroxetine in the form of the hydrochloride and fluvoxamine in the form of the maleate.

As mentioned above, the combination of agomelatine with an SSRI unexpectedly shows a synergistic effect on the central nervous system (CNS). As a consequence, combination therapy using agomelatine and lower doses of the SSRI than normally used in monotherapy, may be effective, and side-effects associated with the larger amounts of SSRI used in monotherapy may be reduced or prevented altogether.

Additionally, combination therapy with agomelatine using a normal dose of SSRI has the advantage that an effective CNS effect may be obtained in the often large number of patients who do not respond to conventional monotherapy with SSRIs.

The amount of agomelatine used in combination therapy may range from about 0.1 to about 150 mg/day, particularly from about 0.1 to about 100 mg/day and more particularly from about 0.5 to about 50 mg/day and even more particularly from about 1 to about 5 mg/day.

SSIRs, including the SSRIs specifically mentioned hereinabove, differ both in molecular weight and in activity. As a consequence, the amount of SSRI used in combination therapy depends on the nature of said SSRI. In one embodiment of the invention, the SSRI is administered at lower doses than required when the compound is used alone. In another embodiment, the SSRI is administered in normal doses.

To prepare the pharmaceutical compositions of this invention, an appropriate amount of the active ingredient(s), in salt form or base form, is combined in an intimate admixture with a pharmaceutically acceptable carrier, which can take a wide variety of forms depending on the form of preparation desired for administration. These pharmaceutical compositions are desirably in unitary dosage form suitable for administration orally, rectally, percutaneously or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed.

It is especially advantageous to formulate the aforementioned pharmaceutical compositions in dosage unit form for ease of administration and uniformity of dosage. As used in the specification and claims, unit dosage form refers to physically discrete units suitable as unitary dosages, each unit containing a predetermined quantity of active ingredient(s) calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Agomelatine may be administered before, during or after the administration of the SSRI provided that the time between the administration of agomelatine and the administration of the SSRI is such that ingredients are allowed to act synergistically on the CNS. When simultaneous administration of agomelatine and an SSRI is envisaged, a composition containing both SSRI and agomelatine may be particularly convenient. Or, agomelatine and the SSRI may be administered separately in the form of suitable compositions. The compositions may be prepared as described hereinabove.

The present invention also comprises products containing agomelatine and an SSRI as a combination preparation for simultaneous, separate or sequential use in psychiatric drug therapy. Such products may comprise, for example, a kit comprising discrete unit dosage forms containing agomelatine and discrete unit dosage forms containing an SSRI, all contained in the same container or pack, e.g. a blister pack.

## Claims

1. Use of agomelatine or a pharmaceutically acceptable salt thereof and a compound, which is a selective serotonin reuptake inhibitor for the preparation of a pharmaceutical composition or kit for the treatment of depression, anxiety disorders and other affective disorders, eating disorders such as bulimia, anorexia and obesity, phobias, dysthymia, premenstrual syndrome, cognitive disorders, impulse control disorders, attention deficit hyperactivity disorder and drug abuse.

2. The use according to claim 1 for the preparation of a pharmaceutical composition or kit for the treatment of anxiety disorders, including general anxiety disorder, panic anxiety, obsessive compulsive disorder, acute stress disorder, post trauma stress disorder or social anxiety disorder.

3. The use according to claim 1 for the preparation of a pharmaceutical composition for the treatment of depression.

4. The use according to claims 1 to 3 wherein agomelatine is used in the form of a tartrate salt.

5. The use according to claims 1-4 wherein the selective serotonin reuptake inhibitor is citalopram, escitalopram, fluoxetine, fluvoxamine, sertraline and paroxetine or a pharmaceutically acceptable salt of any of these compounds.

6. The use according to claims 1-5 wherein the pharmaceutical composition prepared is adapted for simultaneous administration of the active ingredients.

7. The use according to claim 6 wherein the active ingredients are contained in the same unit dosage form.

8. The use according to claims 1-5 wherein the pharmaceutical composition prepared is adapted for sequential administration of the active ingredients.

9. The use according to claim 8 wherein the active ingredients are contained in discrete unit dosage forms.

10. A pharmaceutical composition or kit comprising agomelatine or a pharmaceutically acceptable salt thereof and a selective serotonin reuptake inhibitor, and optionally pharmaceutically acceptable carriers or diluents.

11. The pharmaceutical composition or kit according to claim 10 containing agomelatine in the form of the tartrate salt thereof.

12. The pharmaceutical composition or kit according to claims 10-11 wherein the selective serotonin reuptake inhibitor is citalopram, escitalopram, fluoxetine, fluvoxamine, sertraline and paroxetine or a pharmaceutically acceptable salt of any of these compounds.

13. The pharmaceutical composition according to claims 10-12 which is adapted for simultaneous administration of the active ingredients.

14. The pharmaceutical composition according to claim 13 wherein the active ingredients are contained in the same unit dosage form.

15. The pharmaceutical composition or kit according to claims 10-12 which is adapted for sequential administration of the active ingredients.

16. The pharmaceutical composition according to claim 15 wherein the active ingredients are contained in discrete dosage forms.

17. Agomelatine or a pharmaceutically acceptable salt thereof and a selective serotonin reuptake inhibitor for use in the treatment of depression, anxiety disorders and other affective disorders, eating disorders such as bulimia, anorexia and obesity, phobias, dysthymia, premenstrual syndrome, cognitive disorders, impulse control disorders, attention deficit hyperactivity disorder and drug abuse.

18. The product for the use according to claim 17 for treatment of anxiety disorders, including general anxiety disorder, panic anxiety, obsessive compulsive disorder, acute stress disorder, post trauma stress disorder or social anxiety disorder.

19. The product for the use according to claim 17 for the treatment of depression.

20. The product for the use according to claims 17-19 wherein agomelatine is used in the form of a tartrate salt.

21. The product for the use according to claims 17-20 wherein the selective serotonin reuptake inhibitor is citalopram, escitalopram, fluoxetine, fluvoxamine, sertraline and paroxetine or a pharmaceutically acceptable salt of any of these compounds.

22. The product for the use according to claims 17-21 wherein the active ingredients are administrated simultaneously.

23. The product for the use according to claim 22 wherein the active ingredients are contained in the same unit dosage form.

24. The product for the use according to claims 17-21 wherein the active ingredients are adapted for sequential administration.

25. The product for the use according to claim 24 wherein the active ingredients are contained in discrete unit dosage forms.

## Patentansprüche

1. Verwendung von Agomelatin oder eines pharmazeutisch akzeptablen Salzes hiervon und einer Verbindung, die ein selektiver Serotonin-Wiederaufnahmeinhibitor ist, für die Herstellung einer pharmazeutischen Zusammensetzung oder eines Kits zur Behandlung von Depression, Angststörungen und anderen emotionalen Störungen, Essstörungen, wie z.B. Bulimie, Magersucht und Fettsucht, Phobien, Dysthymie, premenstrualem Syndrom, kognitiven Störungen, Impulskontrollstörungen, Aufmerksamkeitsdefizitsyndrom mit Hyperaktivität und Drogen- bzw. Arzneimittelmissbrauch.

2. Verwendung gemäß Anspruch 1 für die Zubereitung einer pharmazeutischen Zusammensetzung oder eines Kits für die Behandlung von Angststörungen, incl. allgemeiner Angststörungen, panischen Angstzuständen, Zwangsstörungen, akuten Stressstörungen, posttraumatischem Stresssyndrom oder sozialer Angststörung.

3. Verwendung gemäß Anspruch 1 für die Zubereitung einer pharmazeutischen Zusammensetzung für die Behandlung von Depression.

4. Verwendung gemäß den Ansprüchen 1 bis 3, worin Agomelatin in der Form eines Tartratsalzes verwendet wird.

5. Verwendung gemäß den Ansprüchen 1 bis 4, worin der selektive Serotonin-Wiederaufnahmeinhibitor Citalopram, Escitalopram, Fluoxetin, Fluvoxamin, Sertralin und Paroxetin oder ein pharmazeutisch akzeptables Salz von irgendeiner dieser Verbindungen ist.

6. Verwendung gemäß den Ansprüchen 1 bis 5, worin die hergestellte pharmazeutische Zusammensetzung für die gleichzeitige Verabreichung der aktiven Inhaltsstoffe eingerichtet ist.

7. Verwendung gemäß Anspruch 6, worin die aktiven Inhaltsstoffe in der gleichen Dosierungseinheitsform enthalten sind.

8. Verwendung gemäß den Ansprüchen 1 bis 5, worin die hergestellte pharmazeutische Zusammensetzung für die sequentielle Verabreichung der aktiven Inhaltsstoffe eingerichtet ist.

9. Verwendung gemäß Anspruch 8, worin die aktiven Inhaltsstoffe in diskreten Dosierungseinheitsformen enthalten sind.

10. Pharmazeutische Zusammensetzung oder Kit, umfassend Agomelatin oder ein pharmazeutisch akzeptables Salz hiervon und einen selektiven Serotonin-Wiederaufnahmeinhibitor, und optional pharmazeutisch akzeptable Träger oder Verdünnungsmittel.

11. Pharmazeutische Zusammensetzung oder Kit gemäß Anspruch 10, enthaltend Agomelatin in der Form seines Tartratsalzes.

12. Pharmazeutische Zusammensetzung oder Kit gemäß Ansprüchen 10 bis 11, worin der selektive Serotonin-Wiederaufnahmeinhibitor Citalopram, Escitalopram, Fluoxetin, Fluvoxamin, Sertralin und Paroxetin oder ein pharmazeutisch akzeptables Salz von irgendeiner dieser Verbindungen ist.

13. Pharmazeutische Zusammensetzung gemäß Ansprüchen 10 bis 12, die für die gleichzeitige Verabreichung der aktiven Inhaltsstoffe eingerichtet ist.

14. Pharmazeutische Zusammensetzung gemäß Anspruch 13, worin die aktiven Inhaltsstoffe in der gleichen Dosierungseinheitsform enthalten sind.

15. Pharmazeutische Zusammensetzung oder Kit gemäß Ansprüchen 10 bis 12, eingerichtet für die sequentielle Verabreichung der aktiven Inhaltsstoffe.

16. Pharmazeutische Zusammensetzung gemäß Anspruch 15, worin die aktiven Inhaltsstoffe in diskreten Dosierungsformen enthalten sind.

17. Agomelatin oder ein pharmazeutisch akzeptables Salz hiervon und ein selektiver Serotonin-Wiederaufnahmeinhibitor zur Verwendung in der Behandlung von Depressionen, Angststörungen und anderen emotionalen Störungen, Essstörungen, wie z.B. Bulimie, Magersucht und Fettsucht, Phobien, Dysthymie, premenstrualem Syndrom, kognitiven Störungen, Impulskontrollstörungen, Aufmerksamkeitsdefizitsyndrom mit Hyperaktivität und Drogen- bzw. Arzneimittelmissbrauch.

18. Produkt für die Verwendung gemäß Anspruch 17 zur Behandlung von Angststörungen, incl. allgemeiner Angststörungen, panischen Angstzuständen, Zwangsstörungen, akuten Stressstörungen, posttraumatischem Stresssyndrom oder sozialer Angststörung.

19. Produkt zur Verwendung gemäß Anspruch 17 zur Behandlung von Depression.

20. Produkt zur Verwendung gemäß den Ansprüchen 17 bis 19, worin Agomelatin in der Form eines Tartratsalzes verwendet wird.

21. Produkt zur Verwendung gemäß den Ansprüchen 17 bis 20, worin der selektive Serotonin-Wiederaufnahmeinhibitor Citalopram, Escitalopram, Fluoxetin, Fluvoxamin, Sertralin und Paroxetin oder ein pharmazeutisch akzeptables Salz von irgendeiner dieser Verbindungen ist.

22. Produkt zur Verwendung gemäß den Ansprüchen 17 bis 21, worin die aktiven Inhaltsstoffe simultan verabreicht werden.

23. Produkt zur Verwendung gemäß Anspruch 22, worin die aktiven Inhaltsstoffe in der gleichen Dosierungseinheitsform enthalten sind.

24. Produkt zur Verwendung gemäß den Ansprüchen 17 bis 21, worin die aktiven Inhaltsstoffe für die sequentielle Verabreichung eingerichtet sind.

25. Produkt zur Verwendung gemäß Anspruch 24, worin die aktiven Inhaltsstoffe in diskreten Dosierungseinheitsformen enthalten sind.

## Revendications

1. Utilisation d'agomélatine ou d'un sel pharmaceutiquement acceptable de celle-ci et d'un composé, qui est un inhibiteur sélectif de recaptage de la sérotonine, pour la préparation d'une composition pharmaceutique ou d'une trousse pour le traitement de la dépression, des troubles de l'anxiété et d'autres troubles affectifs, des troubles de l'alimentation tels que la boulimie, l'anorexie et l'obésité, des phobies, de la dysthymie, du syndrome prémenstruel, des troubles cognitifs, des troubles de la maîtrise des impulsions, du trouble d'hyperactivité avec déficit de l'attention, et de la toxicomanie.

2. Utilisation selon la revendication 1 pour la préparation d'une composition pharmaceutique ou d'une trousse pour le traitement des troubles de l'anxiété, y compris les troubles de l'anxiété en général, l'anxiété panique, les troubles compulsifs obsessionnels, les troubles de stress aigu, les troubles de stress post-traumatique ou les troubles de l'anxiété liée aux relations sociales.

3. Utilisation selon la revendication 1 pour la préparation d'une composition pharmaceutique pour le traitement de la dépression.

4. Utilisation selon les revendications 1 à 3, dans laquelle l'agomélatine est utilisée sous la forme d'un sel tartrate.

5. Utilisation selon les revendications 1 à 4, dans laquelle l'inhibiteur sélectif du recaptage de la sérotonine est le citalopram, l'escitalopram, la fluoxétine, le fluvoxamine, la sertraline et la peroxétine ou un sel pharmaceutiquement acceptable de l'un quelconque de ces composés.

6. Utilisation selon les revendications 1 à 5, dans laquelle la composition pharmaceutique préparée est adaptée à l'administration simultanée des ingrédients actifs.

7. Utilisation selon la revendication 6, dans laquelle les ingrédients actifs sont contenus dans la même forme posologique unitaire.

8. Utilisation selon les revendications 1 à 5, dans laquelle la composition pharmaceutique préparée est adaptée à l'administration successive des ingrédients actifs.

9. Utilisation selon la revendication 8, dans laquelle les ingrédients actifs sont contenus dans des formes posologiques unitaires discrètes.

10. Composition pharmaceutique ou trousse comprenant de l'agomélatine ou un sel pharmaceutiquement acceptable de celle-ci et un inhibiteur sélectif du recaptage de la sérotonine, et éventuellement des véhicules ou diluants pharmaceutiquement acceptables.

11. Composition pharmaceutique ou trousse selon la revendication 10, contenant l'agomélatine sous la forme du sel tartrate de celle-ci.

12. Composition pharmaceutique ou trousse selon les revendications 10 et 11, dans laquelle l'inhibiteur sélectif du recaptage de la sérotonine est le citalopram, l'escitalopram, la fluoxétine, le fluvoxamine, la sertraline et la peroxétine ou un sel pharmaceutiquement acceptable de l'un quelconque de ces composés.

13. Composition pharmaceutique selon les revendications 10 à 12, qui est adaptée à l'administration simultanée des ingrédients actifs.

14. Composition pharmaceutique selon la revendication 13, dans laquelle les ingrédients actifs sont contenus dans la même forme posologique unitaire.

15. Composition pharmaceutique ou trousse selon les revendications 10 à 12, qui est adaptée à l'administration séquentielle des ingrédients actifs.

16. Composition pharmaceutique selon la revendication 15, dans laquelle les ingrédients actifs sont contenus dans des formes posologiques unitaires discrètes.

17. Agomélatine ou sel pharmaceutiquement acceptable de celle-ci et inhibiteur sélectif de recaptage de la sérotonine pour une utilisation dans le traitement de la dépression, des troubles de l'anxiété et d'autres troubles affectifs, des troubles de l'alimentation tels que la boulimie, l'anorexie et l'obésité, des phobies, de la dysthymie, du syndrome prémenstruel, des troubles cognitifs, des troubles de la maîtrise des impulsions, du trouble d'hyperactivité avec déficit de l'attention, et de la toxicomanie.

18. Produit pour l'utilisation selon la revendication 17 pour le traitement des troubles de l'anxiété, y compris les troubles de l'anxiété en général, l'anxiété panique, les troubles compulsifs obsessionnels, les troubles de stress aigu, les troubles de stress post-traumatique ou les troubles de l'anxiété liée aux relations sociales.

19. Produit pour l'utilisation selon la revendication 17 pour le traitement de la dépression.

20. Produit pour l'utilisation selon les revendications 17 à 19, dans lequel l'agomélatine est utilisée sous la forme d'un sel tartrate.

21. Produit pour l'utilisation selon les revendications 17 à 20, dans lequel l'inhibiteur sélectif du recaptage de la sérotonine est le citalopram, l'escitalopram, la fluoxétine, le fluvoxamine, la sertraline et la peroxétine ou un sel pharmaceutiquement acceptable de l'un quelconque de ces composés.

22. Produit pour l'utilisation selon les revendications 17 à 21, dans lequel les ingrédients actifs sont administrés simultanément.

23. Produit pour l'utilisation selon la revendication 22, dans lequel les ingrédients actifs sont contenus dans la même forme posologique unitaire.

24. Produit pour l'utilisation selon les revendications 17 à 21, dans lequel les ingrédients actifs sont adaptés à une administration séquentielle.

25. Produit pour l'utilisation selon la revendication 24, dans lequel les ingrédients actifs sont contenus dans des formes posologiques unitaires discrètes.
